(19) Europäisches Patentamt
European Patent Office
Office européen des brevets

(11) Veröffentlichungsnummer: **0 014 478**
**A1**

(12) # EUROPÄISCHE PATENTANMELDUNG

(21) Anmeldenummer: **80100680.0**

(22) Anmeldetag: **11.02.80**

(51) Int. Cl.[3]: **C 07 D 495/04**
//A61K31/55, (C07D495/04, 333/00, 313/00)

(30) Priorität: **13.02.79 US 11943**

(43) Veröffentlichungstag der Anmeldung: **20.08.80 Patentblatt 80/17**

(84) Benannte Vertragsstaaten: **AT BE CH DE FR GB IT LU NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Lee, Thomas Bing Kin**
**Latourette Road 5**
**Whithouse Station, N.J.(US)**

(54) **Verfahren zur Herstellung von Alkyl-4,10-dihydro-10-oxothieno-(3,2-c)-(1)-benzoxepin-8-acetat.**

(57) Verfahren zur Synthese *in situ* von Alkyl-4,10-dihydro-10-oxothieno-[3,2-c] -[1] -benzoxepin-8-acetat, dadurch gekennzeichnet, daß man

a) 4-(2-Carboxy-3-thienylmethoxy)-phenylessigsäure zu ihrem Di-Säurehalogenid halogeniert,
b) das Di-Säurehalogenid mit einem Aluminiumhalogenid zum 4,10-Dihydro -10-oxothieno-[3,2-c] -[1]-benzoxepin -8-acetyl-halogenid umsetzt und
c) das erhaltene Acetylhalogenid mit einem niederen Alkanol zur gewünschten Verbindung umsetzt.

EP 0 014 478 A1

Croydon Printing Company Ltd.

HOECHST AKTIENGESELLSCHAFT HOE 79/F 114 Dr. LA /Fr

Verfahren zur Herstellung von Alkyl-4,10-dihydro-10-oxo-thieno-/3,2-c7-/1/-benzoxepin-8-acetat

Die vorliegende Erfindung betrifft ein Verfahren zur Synthese von Alkyl-4,10-dihydro-10-oxothieno-/3,2-c7-/1/-benzoxepin-8-acetat.

Verbindungen der Formel

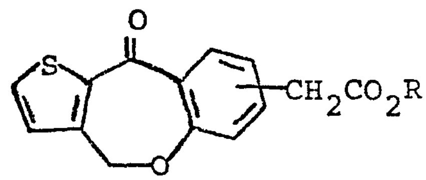

worin R Wasserstoff oder nieders Alkyl bedeutet, sind im US-Patent 4 025 640 beschrieben. Sie zeichnen sich durch entzündungshemmende und analgetische Eigenschaften aus. Weiterhin sind diese Verbindungen im japanischen Patent 61607/1975 beschrieben. Nachfolgend seien zwei mögliche Methoden zur Synthese der unten als Beispiele aufgeführten Verbindungen, gemäß den beiden obigen Patenten, angeführt:

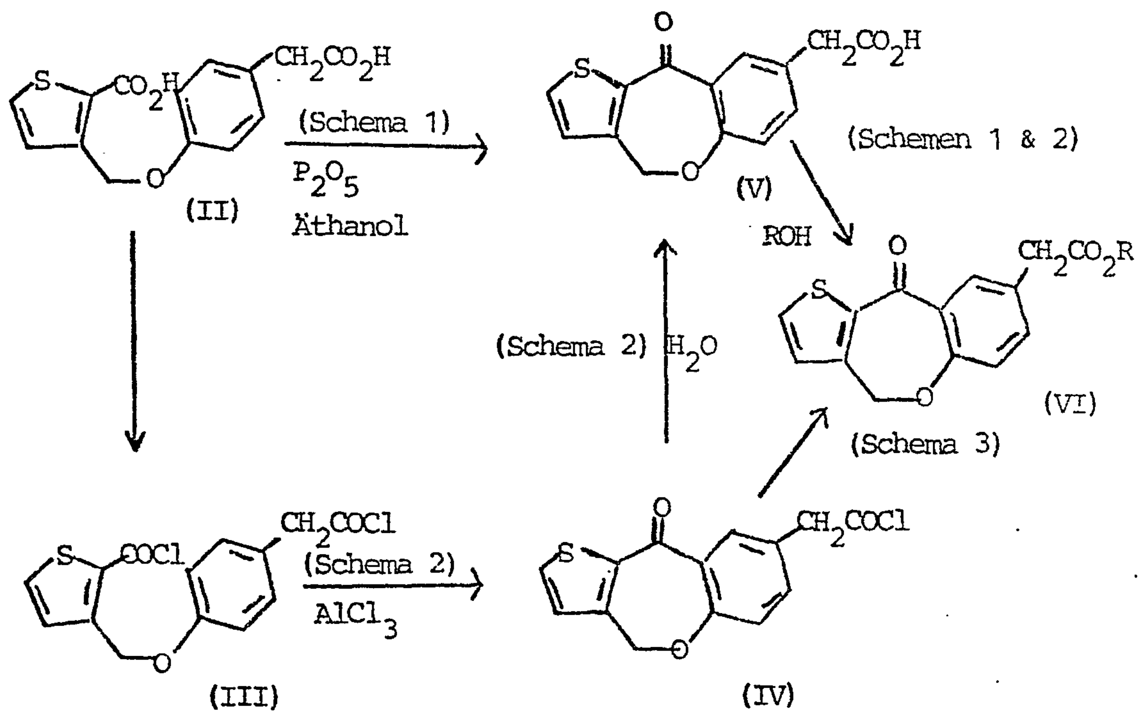

Sowohl im Schema 1 als auch im Schema 2 des obigen Reaktionsablaufs wird als Zwischenprodukt die Verbindung V isoliert und nachfolgend gesondert verestert.

Bei Versuchen, die Gesamtausbeute an Alkyl-4,10-dihydro-10-oxothieno-/3,2-c7-/17-benzoxepin-8-acetat, der Verbindung VI, zu verbessern, wurde nun gefunden, daß überraschenderweise Fortschritte erzielt werden können, wenn die verschiedenen Reaktionsschritte bei der Synthese der obigen Verbindung in situ (Schema 3) erfolgen, wobei die Bildung und Isolierung der Verbindung V als Zwischenprodukt entfallen. So wird erfindungsgemäß die Anfangsverbindung II mit einem geeigneten Reagenz wie $SOCl_2$ oder $SOBr_2$ in das Acylhalogenid überführt, durch Zugabe eines Friedel-Crafts-Katalysators, insbesondere Aluminiumchlorid oder Aluminiumbromid, cyclisiert, und dann gibt man einen niederen Alkohol in den Reaktor, ohne daß sich durch Hydrolyse das Zwischenprodukt V bildet. Diese Schritte erfolgen bei einer Temperatur von etwa 0 bis etwa 25°C, vorzugsweise von etwa 5 bis 15°C, in einem geeigneten Lösungsmittel, vorzugsweise in einem halogenierten Lösungsmittel wie Methylen oder 1,2-Dichloräthan. Die Zugabe eines Katalysators wie Schwefelsäure für die Veresterung ist nicht erforderlich. Zu bemerken wäre jedoch, daß die Veresterung an dem Acylhalogenid und nicht an der freien Carbonsäure V erfolgt und daß weiterhin das Aluminiumchlorid oder -bromid in das entsprechende Aluminium-alkoxid überführt wird, wobei die Alkoxidgruppe bis zu 5 Kohlenstoffatome haben kann.

Ein Vorteil der erfindungsgemäßen Methode besteht darin, daß die Gesamtausbeute des Endproduktes VI aus der Anfangsverbindung II nicht nur gegenüber der bereits bekannten Methode aus Schema 2, sondern auch gegenüber der bekannten Methode des Schemas 1, beträchtlich erhöht wird. Dies ist überraschend, angesichts der Tatsache, daß nacheinander drei Reaktionen, von denen jede in der Lage wäre, eine Reihe

von Nebenreaktionen einzugehen, in einem Reaktionsgefäß vereinigt werden.

Als weiterer Vorteil des erfindungsgemäßen Verfahrens wäre seine Einfachheit, im Vergleich zu den bekannten Schemen 1 und 2 zu nennen, da die Bildung und Isolierung des Zwischenproduktes V fortfallen und da alle aufeinanderfolgenden Reaktionsschritte in situ ablaufen.

Schließlich wäre ein Vorteil des erfindungsgemäßen Verfahrens darin zu sehen, daß der Aluminiumkatalysator nicht verworfen wird, sondern durch Filtrieren und Waschen mit einem entsprechenden Lösungsmittel wieder als Aluminium-alkoxid zurückgewonnen wird. Diese einfache Zurückgewinnung von Aluminium-alkoxid ist äußerst vorteilhaft nicht nur aus wirtschaftlichen Gründen und aus Gründen des Umweltschutzes, sondern auch aufgrund der Tatsache, daß die Gesamtkosten für die Behandlung von Abfallwasser dadurch reduziert werden, daß die Aluminiumsalze im wesentlichen daraus entfernt werden. Bei Verwendung von Isopropylalkohol schließlich wird der Aluminium-Katalysator in Form von Aluminium-isopropoxid, einem wertvollen Handelsprodukt für verschiedenechemische Anwendungsgebiete, insbesondere für Synthesezwecke, z.B. bei Meerwein-Ponndorf-Verley-Reduktionen, zurückgewonnen.

Die Erfindung wird an folgendem Beispiel näher erläutert:

Beispiel

Man erhitzt eine Mischung aus 292,3 g 4-(2-Carboxy-3-thienyl-methoxy)-phenylessigsäure, 500 ml Methylenchlorid oder 1,2-Dichloräthan, 182,3 ml Thionylchlorid und 5 ml N,N-Dimethylformamid 15 Minuten unter Rühren auf 28 - 30°C und hält sie 5 1/2 Stunden bei dieser Temperatur. Dann erhitzt man das Reaktionsgemisch eine Stunde lang am Rückfluß (Temperatur des Reaktionsgefäßes 40°C) und läßt sie

dann 16 Stunden bei Zimmertemperatur stehen.

Dann gibt man wieder 500 ml Methylenchlorid oder 1,2-Dichloräthan zu obiger Lösung, kühlt in einem kalten Wasserbad auf 9 bis 10°C und gibt dann portionsweise 160 g Aluminiumchlorid zu, so daß eine Temperatur des Reaktionsgemisches von 10 bis 15°C nicht überschritten wird. Nach beendeter Zugabe rührt man die Mischung 2 1/2 Stunden bei 10 bis 12°C und kühlt anschließend auf 0 bis 5°C ab. Dann läßt man langsam innerhalb von 1 1/4 Stunden Isopropylalkohol einfließen, wobei die Temperatur unter 15°C gehalten wird. Nach beendeter Zugabe erwärmt man das Reaktionsgemisch 50 Minuten auf 20°C. Das erhaltene Aluminiumisopropoxid wird abfiltriert und mit 500 ml Methylenchlorid gewaschen. Dann extrahiert man das Filtrat nacheinander mit kalter 1-N-Salzsäure (0-5°C), einer Lösung aus 5 %igem Natriumbicarbonat und 5 %igem Natriumchlorid und schließlich mit einer 5 %igen Natriumchloridlösung. Die organische Schicht wird mit 50 g wasserfreiem Magnesiumsulfat getrocknet und filtriert. Das Magnesiumsulfat wird mit 120 ml Methylenchlorid gewaschen. Die vereinigten Filtrate werden unter vermindertem Druck zu einem Rückstand eingedampft, der aus Cyclohexan umkristallisiert wird, wobei man 221 g Isopropyl-4,10-dihydro-10-oxothieno-[3,2-c]-[1]-benzoxepin-8-acetat mit einem Schmelzpunkt von 92 - 94°C erhält, was einer Ausbeute von 70 % entspricht.

Analyse:

| | | |
|---|---|---|
| Berechnet für $C_{17}H_{16}O_4$ | 64,54 % C ; | 5,10 % H. |
| Gefunden: | 64,42 % C ; | 5,17 % H. |

Im Gegensatz dazu erhält man gemäß dem in den Beispielen 1b und 3 des US-Patents 4 025 640 beschriebenen Verfahren, in dem 4,10-Dihydro-10-oxothieno-[3,2-c]-[1]-benzoxepin-8-essigsäurekristalle in 40 %iger Ausbeute hergestellt und abgetrennt werden und nachfolgend in 30 %iger Ausbeute zum

Isopropylester verestert werden, das gewünschte Produkt in einer Gesamtausbeute von nur 12 %.

Patentansprüche:

1. Verfahren zur Synthese in situ von Alkyl-4,10-dihydro-10-oxothieno-[3,2-c]-[1]-benzoxepin-8-acetat, dadurch gekennzeichnet, daß man
   a) 4-(2-Carboxy-3-thienylmethoxy)-phenylessigsäure zu ihrem Di-Säurehalogenid halogeniert,
   b) das Di-Säurehalogenid mit einem Aluminiumhalogenid zum 4,10-Dihydro-10-oxothieno-[3,2-c]-[1]-benzoxepin-8-acetyl-halogenid umsetzt und
   c) das erhaltene Acetylhalogenid mit einem niederen Alkanol zur gewünschten Verbindung umsetzt.

2. Verfahren gemäß Anspruch 1, dadurch gekennzeichnet, daß man als niederen Alkanol einen $C_{1-5}$-Alkanol verwendet.

3. Verfahren gemäß Anspruch 2, dadurch gekennzeichnet, daß man als niederen Alkanol Isopropylalkohol verwendet.

4. Verfahren gemäß Anspruch 1, dadurch gekennzeichnet, daß man als Aluminiumhalogenid Aluminiumchlorid einsetzt.

5. Verfahren gemäß Anspruch 1, dadurch gekennzeichnet, daß man als Halogeniermittel Thionylchlorid verwendet.

6. Verfahren gemäß Anspruch 1, dadurch gekennzeichnet, daß der Reaktionsschritt a) in Methylenchlorid oder 1,2-Dichloräthan als halogeniertem Lösungsmittel durchgeführt wird.

7. Verfahren gemäß Anspruch 1, dadurch gekennzeichnet, daß man als Aluminiumhalogenid Aluminiumbromid verwendet.

8. Verfahren gemäß Anspruch 1, dadurch gekennzeichnet, daß man als Halogeniermittel Thionylbromid verwendet.

Europäisches Patentamt

# EUROPÄISCHER RECHERCHENBERICHT

Nummer der Anmeldung

EP 80 10 0680

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| D | US - A - 4 025 640 (HOECHST) | 1 | C 07 D 495/04// A 61 K 31/55 (C 07 D 495/04 333/00 313/00) |
| D | PATENTS ABSTRACTS OF JAPAN, Band 1, Nr. 17, 23. März 1977, Seite 1103 C 76<br>& JP - A - 51 136 697 (DAIICHI SEIYAKU) 26-11-1976<br>---- | 1 | |

RECHERCHIERTE SACHGEBIETE (Int. Cl. ³)

C 07 D 495/04
A 61 K 31/55

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

✓ Der vorliegende Recherchenbericht wurde für alle Patentansprüche erstellt.

| Recherchenort | Abschlußdatum der Recherche | Prüfer |
|---|---|---|
| Den Haag | 08-05-1980 | ALFARO |